(19) Europäisches Patentamt
European Patent Office
Office européen des brevets

(11) Publication number: **0 373 613 B1**

(12) # EUROPEAN PATENT SPECIFICATION

(45) Date of publication of patent specification: **08.02.95**

(51) Int. Cl.[6]: **C07D 213/78**, C11D 3/39

(21) Application number: **89122974.2**

(22) Date of filing: **12.12.89**

The file contains technical information submitted after the application was filed and not included in this specification

(54) **Pyridine-3-percarboxylic acid monopersulfate.**

(30) Priority: **13.12.88 IT 2292388**

(43) Date of publication of application:
**20.06.90 Bulletin 90/25**

(45) Publication of the grant of the patent:
**08.02.95 Bulletin 95/06**

(84) Designated Contracting States:
**AT BE CH DE ES FR GB LI NL SE**

(56) References cited:
**EP-A- 0 300 461**
**EP-A- 0 340 755**
**US-A- 2 949 468**

**Ullmanns Encyklopädie der technischen Chemie, 4th Edition, vol. 17, pages 720-721**

(73) Proprietor: **AUSIMONT S.p.A.**
**31, Foro Buonaparte**
**I-20100 Milano (IT)**

(72) Inventor: **Venturello, Carlo, Dr.**
**135, Via 23 Marzo**
**I-28100 Novara (IT)**
Inventor: **Cavallotti, Claudio**
**2, Via Lorenteggio**
**I-20146 Milan (IT)**

(74) Representative: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

EP 0 373 613 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to pyridine-3-percarboxylic acid monopersulfate having the formula (I):

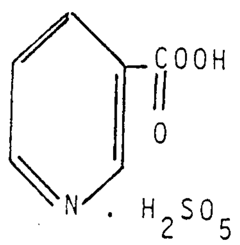

(I)

and, moreover, its preparation and the use thereof as bleaching agent.

Pyridine-3-percarboxylic acid monopersulfate represents a product which is highly interesting from an industrial point of view, particularly due to its high content of active oxygen per weight unit.

More specifically, pyridine-3-percarboxylic acid monopersulfate can be employed in a particularly efficient manner, e.g., in the field of bleaching, in the industry of detergence.

In the past few years organic peracids have attracted increasing interest in the industrial field, particularly due to their excellent employability as bleaching agents in formulations for medium-low temperature washing and, even more important, also due to energy-saving considerations.

Therefore the research activity is aimed at finding organic peracid compounds endowed with the required properties of high bleaching activity, thermal stability and storage stability (shelf life).

Therefore, many (cyclo)aliphatic mono- or di-percarboxylic acids are already known and used, among others, in the detergent field.

From "Ullmanns Encyklopädie der technischen Chemie" (4. Edition, vol.17, pages 720-721) it is furthermore known to use monopersulfate salts as bleaching agents optionally together with halide ion or $\alpha,\beta$ unsaturated carboxylic acids.

Already described percarboxylic acids are, e.g., diperdodecanedioic acid, monoperphthalic acid, diperazelaic acid and substituted diperglutaric and adipic acids, etc.

The conventional percarboxylation processes comprise the oxidation of the substrate with a solution of hydrogen peroxide in concentrated $H_2SO_4$.

The strong acidity of the reaction medium and the presence of a salifiable nitrogen atom of basic character in the substrate used as starting material renders said substrate highly soluble in said acidic medium, making it impossible to apply any of the conventional processes for the isolation of the resulting percarboxylic acid derivative, such as precipitation by dilution with water or extraction with an organic solvent selective for the percarboxylic acid product and not miscible with the remaining reaction mixture.

Surprisingly, it has now been found that the pyridine-3-percarboxylic acid monopersulfate having the above formula (I), salified on the nitrogen atom with persulfuric acid, may be obtained by means of a novel process which is also a part of the present invention.

One object of the present invention is to provide, as per se novel compound, pyridine-3-percarboxylic acid monopersulfate having the above formula (I).

Another object is to provide a simple and inexpensive process for the preparation of the monopersulfate of formula (I).

A further object is the use of 3-pyridine-percarboxylic acid monopersulfate as bleaching agent in detergent formulations, especially those intended for low-medium temperature use.

These and still other objects which will become even clearer for those skilled in the art from the following detailed disclosure, are achieved, according to the present invention, by 3-pyridine-percarboxylic acid monopersulfate and the process for the preparation thereof wherein nicotinic acid (pyridine-3-carboxylic acid) or its N-sulfate salt is reacted with $H_2O_2$ in concentrated $H_2SO_4$ and the resulting monopersulfate (I) is then separated from the reaction mixture by the addition of ethyl acetate.

In this way the compound of formula (I) is obtained as a crystalline solid through its insolubilization in the reaction medium by ethyl acetate.

More specifically, the process according to the present invention comprises the percarboxylation reaction of pyridine-3-carboxylic acid or the N-sulfate salt thereof in an acid medium of concentrated $H_2SO_4$ with $H_2O_2$ and the subsequent addition, at the end of the process, of ethyl acetate.

This leads to the separation of the pyridine-3-percarboxylic acid monopersulfate in a stable solid form.

As mentioned above, the pyridine-3-carboxylic acid substrate used as the starting material may optionally already be salified as sulfate on the nitrogen atom.

The substrate used as the starting material is a per se known compound (nicotinic acid).

The obtained product is then filtered off, washed with the solvent, dried etc., according to per se known techniques.

According to a preferred embodiment, the percarboxylation of pyridine-3-carboxylic acid or its N-sulfate is carried out by gradually adding $H_2O_2$ (having a concentration of from 70% to 90% by weight) to a solution of the substrate in concentrated $H_2SO_4$ (96-98%), maintaining the reaction temperature throughout the reaction within the range of from 0 to 25 °C.

It has been found that it is also possible to prepare in advance the salt of the substrate, in the form of the N-sulfate, by working under the same conditions as described above, but in the absence of $H_2O_2$ and by separating the salt thus obtained which then is peroxidized.

The amount of $H_2SO_4$, determined at a concentration of 100%, preferably is substantially equal to 9 moles per mole of substrate.

The hydrogen peroxide is used in an amount which is in excess with respect to the substrate, substantially equal to 5.5 moles per mole of substrate.

The amount of ethyl acetate used suitably is at least equal to 4 liters/per mole of substrate and, furthermore, is advantageously added at a temperature not higher than 0 °C.

The pyridine-3-percarboxylic acid monopersulfate usually is solid at room temperature. It may be especially useful in formulations of detergent compositions, e.g., granular formulations, as bleaching agents in solution over a wide temperature range.

The detergent compositions may be formulated according to conventional methods, together with other components and/or additives, etc.

The present invention will now be described in still more detail in the following examples, which are given for purely illustrative purposes.

The pyridine-3-percarboxylic acid monopersulfate was characterized by elemental analysis, by determining tis content of active oxygen (by iodometric titration), and by using Fourier Transform Infrared Spectroscopy (FT-IR).

## EXAMPLE 1

59.6 g (0.583 mole) of $H_2SO_4$ (96%) were placed in a beaker equipped with stirrer, thermometer, and external bath.

The internal temperature was brought to 0 °C and 14.4 g (0.36 mole) of $H_2O_2$ at 85% were slowly added under stirring, maintaining the temperature below 5 °C.

8 g (0.0650 mole) of pyridine-3-carboxylic acid (nicotinic acid) were then gradually added and the reaction was continued for 4 hours at 20 - 25 °C.

At the end, the reaction mixture was poured into 250 ml of ethyl acetate maintained under stirring at between -10 ° and 0 °C.

After 2 hours of reaction at this temperature, the crystalline product was filtered, under vacuum, over a porous septum. The product was directly washed on the filter with ethyl acetate (50 ml) and ethyl ether (50 ml) and then dried over $CaCl_2$ under vacuum and at room temperature for 1 hour.

Obtained were 12.5 g of crystalline product having an active oxygen content of 11.75% (theoretical value for pyridine-3-percarboxylic acid monopersulfate: 12.64%). Yield 70%.

| Elemental Analysis | |
|---|---|
| Calculated for $C_6H_7O_8NS$: | C = 28.47%; H = 2.78%; N = 5.53%; O (active) = 12.64%; $H_2SO_5$ = 45.07%. |
| Found: | C = 28.90%; H = 2.86%; N = 5.69%; O (active) = 11.75%; $H_2SO_5$ = 42.30%. |
| Melting Point: | 85 °C (with decomposition). |

EXAMPLE 2 (Application example)

Bleaching with pyridine-3-percarboxylic acid monopersulfate

Bleaching tests were carried out with a detergent formulation containing pyridine-3-percarboxylic acid monopersulfate in the amount reported in the following Table 1 (composition A) and a similar comparative formulation containing, as bleaching agent, H 48 peracid (Mg salt of monoperphthalic acid, a known commercial product, manufactured by INTEROX Chemical Ltd London, U.K., for use in the detergent art) (composition B).

Compositions A and B were obtained by dry blending of a detergent base (identical for both compositions) with the above bleaching agents. As detergent base a granular composition was used which contained all of the conventional components of a detergent for washing machines (surfactants, builders, etc.), except the chemical bleaching agents, and was obtained by atomization of the component mixture. The detergent base employed had the following composition:

| | Weight % |
|---|---|
| Total surfactants | 15.4 |
| Sodium alkyl ($C_{12}$) benzenesulphonate, soap, ethoxylated (EO) alcohol ($C_{16}$-$C_{18}$) Total sodium phosphates | 8.8 |
| Zeolite A | 19.8 |
| Silicate ($SiO_2/Na_2O = 2$) | 4.4 |
| Sodium sulphate | 36.6 |
| Sodium carbonate | 6.6 |
| Carboxymethylcellulose | 1.1 |
| Anti-encrusting copolymers | 4.8 |
| Water | 2.2 |
| Optical bleaching agents | 0.3 |

The metering of the compositions A and B was carried out in a way such as to introduce into the washing machine a constant amount of detergent base (corresponding to 120 g) and an amount of bleaching agent such as to introduce into the washing machine a quantity of total initial active oxygen of about 2g of oxygen for each washing cycle, identical for all operations.
Specifically, the compositions A and B given in the following Table 1 were used in the bleaching tests.

TABLE 1

| Composition A | |
|---|---|
| Detergent base | 120 g |
| Pyridine-3-percarboxylic acid monopersulfate having 11.1% of active oxygen | 18 g |
| **Composition B** | |
| Detergent base | 120 g |
| H 48 having 5.5% of active oxygen | 39 g |

The tests were carried out in a commercial IGNIS® Mod. 644 washing machine by introducing into the machine two cotton specimens (15 x 15 cm) stained with standard stains or red wine at the EMPA INSTITUTE of St. Gallen (Switzerland) and marked with the "EMPA 114" mark, together with 3 kg of cleaned cotton wipers as ballast for each washing operation.
The washing operations were carried out by using a conventional low temperature program (about 40 °C). Normal tap water, having a hardness of 14 °F, was used.
The results of the tests are reported in the following Table 2, wherein the data are expressed as % Bleaching, defined as:

$$\% \text{ Bleaching} = \frac{A - B}{C - B} \times 100$$

wherein:

A = degree of whiteness (%) of the specimen bleached after the test;
B = degree of whiteness (%) of the specimen before the test;
C = degree of whiteness (%) of the completely bleached specimen.

The degree of whiteness was measured by means of an Elrepho Zeiss Reflectometer, using a filter No. 6 ( $\lambda$ = 464 nm) and assuming MgO = 100% of whiteness.

TABLE 2

| | % Bleaching |
|---|---|
| Composition A | 57 |
| Composition B | 52 |

The data show the higher bleaching power of the claimed peracid in comparison to that of H 48.

EXAMPLE 3 (Application example)

Bleaching tests were carried out with the pyridine-3-percarboxylic acid monopersulfate at an acid pH in comparison to H 48.

The results are listed in the following Table 3.

All tests were carried out at constant temperature of 60 °C with an initial concentration of total active oxygen in the bleaching solution of 200 mg/l, identical for both products.

Process

For each test 500 ml of deionized water, contained in a 1000 ml flask equipped with a condenser, were heated to a temperature of 60 °C.

The bleaching product was dissolved under stirring in the amounts shown in the following Table 3 and immediately thereafter two cotton specimens (10 x 10 cm) stained with standard stains of red wine at the EMPA INSTITUTE of St. Gallen (Switzerland), and marked with the "EMPA 114" mark, were added to the solution having a pH value of 3 - 4.

Thereafter the system was kept stirred for 60 minutes and finally the specimens, rinsed under running water, dried and ironed, were subjected to an evaluation of the bleaching effect by determining the degree of whiteness by reflectometry. The results are reported in the following Table 1, expressed in terms of % Bleaching as defined in example 2 above.

The data in Table 3 show that the acid salt of the present invention, in acid solution, has a particularly high bleaching activity, and a much higher one than H 48. This is particularly surprising in consideration of the fact that peroxy compounds generally show a very modest bleaching activity under these conditions.

TABLE 3

| Tests Carried out at Acid pH (3-4) | | | |
|---|---|---|---|
| Compound | Amount used in the tests (grams) | Initial concentration of total active oxygen (mg/l) | % Bleaching at 60 °C |
| Example 1 (TITER = 11.75% of active oxygen) | 0.86 | 200 | 74.0 |
| H 48 (TITER = 5.5% of active oxygen) | 1.86 | 200 | 60 |

## Claims

1. Pyridine-3-percarboxylic acid monopersulfate having the formula:

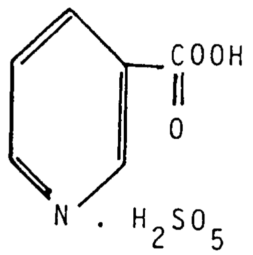

(I)

2. Process for preparing pyridine-3-percarboxylic acid monopersulfate, wherein pyridine-3-carboxylic acid or its N-sulphate salt is reacted in concentrated $H_2SO_4$ with $H_2O_2$ in an amount which is substantially equal to 5,5 moles per mole of pyridine-3-carboxylic acid and the resulting percarboxylic acid salt is separated from the reaction mixture by the addition of ethyl acetate.

3. Process for preparing pyridine-3-percarboxylic acid monopersulfate wherein pyridine-3-carboxylic acid is converted into the corresponding $HSO_4$ salt which is then reacted in concentrated $H_2SO_4$ with $H_2O_2$ in an amount which is substantially equal to 5.5 moles per mole of pyridine-3-carboxylic acid, and the resulting product is separated from the reaction mixture by the addition of ethyl acetate.

4. Process according to any one of claims 2 and 3 wherein the pyridine-3-carboxylic acid or its N-sulfate salt is graduallly reacted in concentrated $H_2SO_4$ and at a temperature of from 0° to 25°C with $H_2O_2$ in an amount of substantially equal to 5.5 moles per mole of pyridine-3-carboxylic acid, the $H_2O_2$ have a concentration of from 70% to 90% by weight.

5. Process according to any one of claims 2 to 4, wherein the amount of $H_2SO_4$ is substantially equal to 9 moles per mole of pyridine-3-carboxylic acid.

6. Process according to any one of claims 2 to 5, wherein the amount of ethyl acetate used is at least equal to 4 litres per mole of pyridine-3-carboxylic acid.

7. Process according to any one of claims 2 to 6, wherein the ethyl acetate is added at a temperature not higher than 0°C.

8. Use of pyridine-3-percarboxylic acid monopersulfate as bleaching agent in detergent formulations.

## Patentansprüche

1. Pyridin-3-percarbonsäure-monopersulfat mit der Formel:

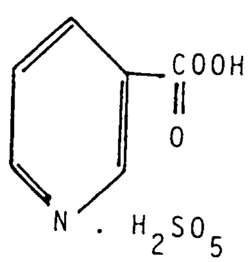

(I)

2. Verfahren zur Herstellung von Pyridin-3-percarbonsäuremonopersulfat, worin Pyridin-3-carbonsäure oder deren N-Sulfatsalz in konzentrierter $H_2SO_4$ umgesetzt wird mit $H_2O_2$ in einer Menge, die im wesentlichen 5,5 Mol pro Mol Pyridin-3-carbonsäure beträgt, und das erhaltene Percarbonsäuresalz von der Reaktionsmischung durch Zugabe von Ethylacetat abgetrennt wird.

3. Verfahren zur Herstellung von Pyridin-3-percarbonsäuremonopersulfat, worin Pyridin-3-carbonsäure in das entsprechende $HSO_4$-Salz umgewandelt wird, das dann in konzentrierter $H_2SO_4$ umgesetzt wird mit $H_2O_2$ in einer Menge, die im wesentlichen 5,5 Mol pro Mol Pyridin-3-carbonsäure beträgt, und das erhaltene Produkt von der Reaktionsmischung durch Zugabe von Ethylacetat abgetrennt wird.

4. Verfahren nach einem der Ansprüche 2 und 3, worin die Pyridin-3-carbonsäure oder deren N-Sulfatsalz in konzentrierter $H_2SO_4$ und bei Temperaturen von 0° bis 25° C allmählich umgesetzt wird mit $H_2O_2$ in einer Menge, die im wesentlichen 5,5 Mol pro Mol Pyridin-3-carbonsäure beträgt, wobei das $H_2O_2$ eine Konzentration von 70 bis 90 Gew.-% hat.

5. Verfahren nach einem der Ansprüche 2 bis 4, worin die Menge an $H_2SO_4$ im wesentlichen 9 Mol pro Mol Pyridin-3-carbonsäure entspricht.

6. Verfahren nach einem der Ansprüche 2 bis 5, worin die Menge an verwendetem Ethylacetat mindestens 4 l pro Mol Pyridin-3-carbonsäure entspricht.

7. Verfahren nach einem der Ansprüche 2 bis 6, worin das Ethylacetat bei einer Temperatur von nicht mehr als 0° C zugegeben wird.

8. Verwendung von Pyridin-3-carbonsäure-monopersulfat als Bleichmittel in Detergentformulierungen.

**Revendications**

1. Monopersulfate de l'acide pyridine-3-percarboxylique, représenté par la formule :

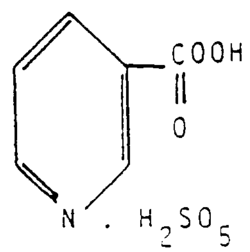

(I)

2. Procédé de préparation du monopersulfate de l'acide pyridine-3-percarboxylique, suivant lequel de l'acide pyridine-3-carboxylique ou son sel N-sulfate est mis à réagir dans $H_2SO_4$ concentré avec $H_2O_2$ dans une quantité qui est sensiblement égale à 5,5 moles par mole d'acide pyridine-3-carboxylique, et le sel d'acide percarboxylique résultant est séparé du mélange réactionnel par l'addition d'acétate d'éthyle.

3. Procédé de préparation du monopersulfate de l'acide pyridine-3-percarboxylique, suivant lequel l'acide pyridine-3-carboxylique est converti en le sel $HSO_4$ correspondant, lequel est ensuite mis à réagir dans $H_2SO_4$ concentré avec $H_2O_2$ dans une quantité qui est sensiblement égale à 5,5 moles par mole de l'acide pyridine-3-carboxylique, et le produit résultant est séparé du mélange réactionnel par l'addition d'acétate d'éthyle.

4. Procédé selon l'une des revendications 2 et 3, suivant lequel l'acide pyridine-3-carboxylique ou son sel N-sulfate est progressivement mis à réagir dans $H_2SO_4$ concentré et à une température de 0° à 25°C avec $H_2O_2$, dans une quantité sensiblement égale à 5,5 moles par mole de l'acide pyridine-3-carboxylique, l'$H_2O_2$ ayant une concentration de 70% à 90% en poids.

**5.** Procédé selon l'une des revendications 2 à 4, dans lequel la quantité d'$H_2SO_4$ est sensiblement égale à 9 moles par mole d'acide pyridine-3-carboxylique.

**6.** Procédé selon l'une des revendications 2 à 5, dans lequel la quantité d'acétate d'éthyle utilisée est au moins égale à 4 litres par mole d'acide pyridine-3-carboxylique.

**7.** Procédé selon l'une des revendications 2 à 6, dans lequel l'acétate d'éthyle est ajouté à une température non supérieure à 0 °C.

**8.** Utilisation du monopersulfate de l'acide pyridine-3-percarboxylique comme agent de blanchiment dans des formulations de détergents.